# EUROPEAN PATENT SPECIFICATION

(11) **EP 1 044 294 B1**
(45) Date of publication and mention of the grant of the patent: **07.12.2005**
(21) Application number: 98964904.1
(22) Date of filing: 24.12.1998
(51) Int. Cl.: D01F 6/36, D04H 3/02, A61L 15/00

(54) **METHOD FOR PREPARING NON-WOVEN WEB OF SUPERABSORBENT FIBER**
VERFAHREN ZUR HERSTELLUNG VON VLIESSTOFFEN AUS HOCHABSORBIERENDER FASER
PROCEDE DE PREPARATION DES BANDES NON TISSEE A PARTIR DE FIBRE SUPERABSORBANTE

(30) Priority: 31.12.1997 US 70212 P
(43) Date of publication of application: 18.10.2000
(73) Proprietor: KIMBERLY-CLARK WORLDWIDE, INC., Neenah, Wisconsin 54956 (US)
(72) Inventor: QIN, Jian, Appleton, WI 54915 (US); LI, Yong, Appleton, WI 54915 (US); VAN DYKE, Wendy, Lynn, Neenah, WI 54956 (US); WISNESKI, Anthony, John, Kimberly, WI 54136 (US); WALLAJAPET, Palani, Raj, Ramaswami, Wauwatosa, WI 53225 (US); RHIM, Hannong, Roswell, GA 30075 (US)
(74) Representative: Davies, Christopher Robert
(86) International application number: PCT/US1998/027509
(87) International publication number: WO 1999/034041

(56) References cited:
- EP-A- 0 268 498
- EP-A- 0 547 604
- EP-B- 0 656 077

## Description

This invention relates to a nonwoven web of superabsorbent fiber. In one aspect, this invention relates to a method of preparing a nonwoven web of superabsorbent fine fiber.

Certain polymers are termed superabsorbent polymers for their ability to take up and hold fluids. Poly(acrylic acid) copolymer is one example of such a superabsorbent polymer.

Dry spinning can form superabsorbent polymer into continuous filaments. Dry spinning extrudes an aqueous solution of the polymer into air. Using a highly concentrated polymer solution, liquid filaments are extruded and then solidified, dried, hot-drawn, and heat-treated in a gaseous environment.

A nonwoven superabsorbent fibrous web can be produced by first forming an aqueous fiber-forming polymer solution into filaments which are contacted with a primary air stream having a velocity sufficient to attenuate the filaments. The attenuated filaments are contacted in a fiber-forming zone with a secondary air stream having a velocity effective to attenuate the filaments further, to "fragment" the filaments into fibers, and to transport the fibers to a web-forming zone. The "fragmented" fibers are collected in a reticulated web formed in the web-forming zone, and the web is cured.

A nonwoven fabric of water-soluble resin fibers can consist of water-soluble resin fine fibers having a mean fiber diameter of 30 µm or less and a basis weight of 5 to 500 g/m². The fabric can be produced by extruding an aqueous solution of water-soluble resin or a water-soluble resin melt plasticized with water through nozzles, stretching the extruded material to form fibers by a high speed gas flow, heating the fibers to evaporate the water in the fibers, and then collecting the fibers. The water-soluble resins can include poly(vinyl alcohol) when the application is directed primarily to the use of pullulan, a natural glucan. The high speed gas flow can consist of air at a temperature of from 20°C to 60°C at a linear velocity of 10 to 1,000 m/sec. The fibers can be dried by banks of infrared heaters located on both sides of and parallel to the fiber stream.

Some methods of forming fibrous webs or products from a solution of a polymer or molten polymer produce very short fibers and, consequently, differ significantly from melt-blowing or spunbonding processes which can be used to prepare nonwoven webs from molten thermoplastic polymers.

Steam can be used in the fiber-forming process. A water-containing polymeric composition can be extruded under conditions using a supercritical fluid solution, preventing flashing, and spraying water-imbibed gelled fibers to form webs.

Meltblowing can be used in the fiber-forming process.

Coforming can be used in the fiber-forming process. Fibers or particles are commingled with meltblown fibers as they are formed.

Spunbonding can be used in the fiber-forming process.

A prior art extrusion process for forming superabsorbent fibers is disclosed in EP0547604.

Superabsorbent precursor polymers having high molecular weights, e.g. by way of example, molecular weights higher than 500,000, and minimum cross linkage can provide high fluid absorbency under load.

By superabsorbent polymer is meant a polymer which can provide high fluid absorbency under load at a level of 10 grams of 0.9% by wt. aqueous sodium chloride per gram of dry absorbent fiber or nonwoven web.

Spinning fiber from high molecular weight polymers is very challenging, even in the case where the polymer is a linear chain polymer, particularly when the molecular chain is flexible.

Ultra high modulus and high strength fiber from extremely high molecular weight polyethylene are prepared only by a slow gel spinning.

Fiber spinning from a solution of a linear chain, flexible polymer involves un-entangling and stretching of coiled and entangled polymer molecules in the solution. When these molecules are large, the process of un-entangling and stretching becomes very difficult and slow, if successful at all. The relaxation time is long.

Accordingly, preparing substantially continuous fiber from a solution of high molecular weight polymer has been thought to be impossible particularly with high speed nonwoven spinning processes. The high speed nonwoven spinning process is operated at spinning speeds 10 times to 100 times higher than in the conventional textile fiber spinning. At the higher spinning speeds, microfiber web from high molecular weight (124,000 - 180,000) poly(vinyl alcohol) was observed to become shoty, indicating fiber breakage.

It is an object of the present invention to provide a method of preparing a significantly improved nonwoven web including substantially continuous superabsorbent microfiber having mechanical strength, high fluid absorbency, and preferred handling properties.

It is an object of the present invention to provide a method of preparing a novel and significantly improved nonwoven web including continuous superabsorbent fine fiber having mechanical strength, high fluid absorbency, and preferred handling properties.

These and other objects will become apparent further to one having ordinary skill in the art from a consideration of the detailed description of the specification and claims which follow.

Briefly, the present invention provides a method of preparing a nonwoven web of substantially continuous superabsorbent fine fiber.

According to one aspect of the present invention there is provided a method of providing a nonwoven web as claimed in claim 1.

An aqueous polymer solution is prepared composed of about 10 to 75 percent by weight of a linear superabsorbent precursor polymer having a molecular weight of from about 300,000 to about 10,000,000. The polymer solution is extruded at a temperature in the range about 20°C to about 180°C, at a viscosity in the range of about 3 to about 1000 Pa sec, through a die having a plurality of orifices to form a plurality of threadlines. The die orifices have diameters in the range of about 0.20 to about 1.2 mm. The resulting threadlines are attenuated with a primary gaseous source under conditions sufifcient to permit the viscosity of each threadline, as it leaves a die orifice and for a distance of no more than about 8 cm, to increase incrementally with increasing distance from the die, while substantially maintaining uniformity of viscosity in the radial direction, at a rate sufficient to provide fiber having the desired attenuation and mean fiber diameter without significant fiber breakage. The primary gaseous source has a relative humidity of from about 30 to 100 percent, a temperature of from about 20°C to about 100°C, a velocity of from about 150 to about 400 m/s, a horizontal angle of incidence of from about 70° to about 110°, and a vertical angle of incidence of no more than about 90°. The threadlines are dried to form fibers with a secondary gaseous source at a temperature of from about 140°C to about 320°C and a velocity of from about 60 to about 125 m/s. The secondary gaseous source has a horizontal angle of incidence of from about 70° to about 110° and a vertical angle of incidence of no more than about 90°. The fibers are deposited randomly on a moving foraminous surface to form a substantially uniform web on a scale of from about 0.4 to about 1.9 cm². The moving foraminous surface is positioned about 10 to about 60 cm from the opening from which the last gaseous source to contact the threadlines emerges. The fibers have a mean fiber diameter in the range of from about 0.1 to about 10 µm and are substantially free of shot. The attenuating and drying steps are carried out under conditions of controlled macro scale turbulence, and the fibers are of a length such that they can be regarded as continuous in comparison with their diameters. The uniform web is exposed to a high energy source selected from the group consisting of heat, electron beam, microwave, and radio frequency irradiation to insolubilize the polymer and render a stable cross-link in the superabsorbent precursor polymer. The stabilized web is post treated for certain web structure and attributes, such as humidifying, compacting, embossing, bonding, and laminating.

A novel nonwoven web and method of preparing a significantly improved nonwoven is further disclosed web including continuous superabsorbent fine fiber in which the primary gaseous source has a relative humidity of from about 60 to 95 percent, a temperature of from about 20°C to about 100°C, a velocity of from about 30 to about 150 m/s, a horizontal angle of incidence of from about 70° to about 110°, and a vertical angle of incidence of no more than about 90°. The threadlines are dried to form fibers with a secondary gaseous source at a temperature of from about 140°C to about 320°C and having a velocity of from about 30 to about 150 m/s at a horizontal angle of incidence of from about 70° to about 110°, and a vertical angle of incidence of no more than about 90°. The fibers are deposited randomly on a moving foraminous surface to form a substantially uniform web on a scale of from about 1.9 to about 6.5 cm², the moving foraminous surface being from about 10 to about 100 cm from the opening from which the last gaseous source to contact the threadlines emerges, which fibers have a mean fiber diameter in the range of from about 10 to about 30 µm and are substantially uniform in diameter. The attenuating and drying steps are carried out under conditions of minimal macro scale turbulence.

A novel nonwoven web and method of preparing a significantly improved continuous superabsorbent fine fibers and nonwoven web is further disclosed including these fibers in which the primary gaseous source has a relative humidity of from about 65 to 90 percent, a temperature of from about 20°C to about 100°C, a velocity of less than about 30 m/s, a horizontal angle of incidence of from about 70° to about 110°, and a vertical angle of incidence of about 90°. The threadlines are dried to form fibers with a secondary gaseous source at a temperature of from about 140°C to about 320°C and having a velocity of less than about 30 m/s, a horizontal angle of incidence of from about 70° to about 110°, and a vertical angle of incidence of about 90°. The resulting fibers are attenuated with a tertiary gaseous source having a temperature of in the range of about 10°C to about 50°C, a velocity in the range of about 30 to about 240 m/s, a horizontal angle of incidence of from about 70° to about 110°, and a vertical angle of incidence of no more than about 90°. The fibers are deposited randomly on a moving foraminous surface to form a substantially uniform web on a scale of from about 1.9 to about 6.5 cm², the moving foraminous surface being positioned at about 10 to about 100 cm from the opening from which the last gaseous source to contact the threadlines emerges, which fibers have a mean fiber diameter in the range of from about 10 to about 30 µm and are substantially uniform in diameter, in which the conditioning, drying, and attenuating steps are carried out under conditions of minimal macro scale turbulence.

Significantly improved substantially continuous superabsorbent microfiber and a nonwoven web including these fibers are further disclosed, in which the fibers have a mean fiber diameter in the range of from about 0.1 to about 10 µm, are substantially free of shot, and are of a length such that they can be regarded as continuous in comparison with their diameters. The web is substantially uniform on a scale of from about 0.4 to about 1.9 cm², depending on the mean fiber diameter.

A significantly improved nonwoven web including continuous superabsorbent fine fiber is further disclosed, in which the fibers have a mean fiber diameter in the range of from about 10 to about 100 µm, are essentially free of shot, and are substantially uniform in diameter; and the web is substantially uniform on a scale of from about 1.9 to about 6.5 cm², depending on the mean fiber diameter.

A disposable absorbent product having a significantly improved nonwoven web including substantially continuous or continuous superabsorbent fiber is also disclosed.

The superabsorbent fiber nonwoven webs of the present invention are particularly useful in the production of such disposable absorbent products as diapers; training pants; catamenial devices, such as sanitary napkins, tampons, and the like; incontinent products; wipes; and the like.

Various preferred embodiments of the present invention will now be described by way of example only and with reference to the accompanying drawings, in which:
Figure 1 is a perspective schematic view partially illustrating the preparation of a nonwoven web in accordance with one embodiment of the present invention and illustrating the horizontal angle of incidence.
Figure 2 shows a cross-section view of the lower part of the die tip portion of the die of Figure 1, taken along line 2-2. The figure illustrates the vertical angle of incidence.
Figure 3 is a perspective view of a portion of a superabsorbent threadline produced in accordance with a preferred embodiment of the present invention.
Figure 4 is a perspective view of a portion of the threadline shown in Figure 3.

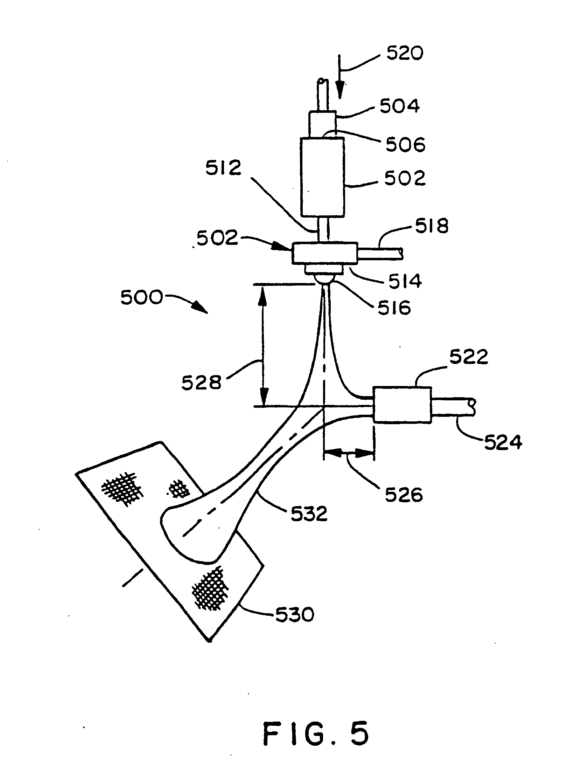

Figure 5 is a schematic representation of one embodiment of the present invention.

In has been found through empirical development that high absorbency nonwoven webs including substantially continuous fiber, i.e., having very little "shots," were obtained through a high speed nonwoven spinning process, with novel process modification, from extremely high molecular weight superabsorbent precursor polymers as high as 8,000,000.

The mechanism of the novel fiber forming is believed to involve a strong affinity of water molecules toward the carboxyl group of sodium polyacrylic acid copolymer which, for example, may render the long polymer chain stiffer, thereby facilitating un-entangling and stretching. The mechanism may involve ionic repairing of the carboxyl group of the sodium polyacrylic acid copolymer.

A substantially improved nonwoven web has been prepared from sodium polyacrylic acid copolymer under a novel process including a meticulous control of the gaseous environment, into which solution threadlines are extruded, in humidity and temperature, preventing premature excessive evaporation of solvent water before wet threadlines are attenuated into a desirable fine size without breakage or "fragmentation." The substantially continuous fiber contained very little "shot," and the webs were very soft and uniform particularly when turbulence of the primary steam and the secondary hot drying airs were controlled.

"Web uniformity" is a term which is used herein to refer to the extent to which any portion of a nonwoven web produced in accordance with the present invention having a given area is like any other portion having the same area. Web uniformity is a function of fiber diameter and the manner in which fibers are deposited on the moving foraminous surface. Ideally, any given area of the web will be indistinguishable from any other area with respect to such parameters as porosity, void volume, pore size, web thickness, and the like. However, uniformity variations are manifest in webs as portions which are thinner than other portions. Such variations can be estimated visually to give a subjective determination of uniformity. Alternatively, web uniformity can be qualitatively estimated by measuring web thickness or light transmission through the web.

The term "relatively small scale" is used throughout this specification in reference to web uniformity and defines the approximate area of each of several portions of the web which are to be compared. In general, the scale typically will be in the range of from about 0.4 to about 6.5 cm², depending upon the mean fiber diameter. When the mean fiber diameter is 10 µm or less, the appropriate area in cm² for evaluating web uniformity, i.e., the scale, is 0.19 times the mean fiber diameter in µm or 0.4 cm², whichever is greater. The scale is determined by multiplying the mean fiber diameter by 0.19 when the mean fiber diameter is in the range of about 2.1 to about 10 µm. For mean fiber diameters of about 2.1 µm or less, however, the scale is 0.4 cm². When the mean fiber diameter is greater than 10 µm, the appropriate multiplier is 0.215. The phrase "on a scale of from about 0.4 to about 6.5 cm²" means that the area of one portion of a nonwoven web which is to be compared with other portions of the same web, each of which portions has essentially the same area, will be in the range given. The area selected, in cm² will be (1) approximately 0.19 times the mean fiber diameter in µm when the mean fiber diameter is 10 µm or less or 0.4 cm², whichever is greater, or (2) approximately 0.215 times the mean fiber diameter when the mean fiber diameter is greater than 10 µm.

As used herein, the term "shot" refers to particles of polymer which generally have diameters greater than the average diameter of the fibers produced by the extrusion process. The production of shot typically is associated with filament breakage and the accompanying accumulation of polymer solution on the die tip.

The term "molecular weight" refers to weight average molecular weight, unless stated otherwise.

The term "turbulence" is used herein to refer to the departure in a fluid, typically a gas, from a smooth or streamlined flow. The term is meant to apply to the extent or degree to which the fluid flow varies erratically in magnitude and direction with time and is essentially variable in pattern. The term "macro scale turbulence" means only that the turbulence is on a scale such that it affects the orientation and spacing of the fibers or fiber segments relative to each other as they approach the web-forming surface, in which the length of such fiber segments is equal to or less than the scale. Turbulence is "controlled" when its magnitude is maintained below an empirically determined level. The minimal turbulence can be achieved by the proper selection of process variables and is permitted to increase only to an extent necessary to achieve a given objective.

Because of the difficulty of measuring turbulence, an indirect means for determining when turbulence is being controlled to a sufficient degree must be used. Such indirect means is web uniformity. Web uniformity is defined as a function of both the area of the web to be evaluated and the mean diameter of the fibers of which the web is composed. For example, producing nonwoven webs will give a very uniform product if the scale, i.e., the area of the web used for comparison purposes, is large, for example, on the order of several square meters. At the other extreme, uniformity of the same web is very poor if the scale is so small that it is on the order of the mean diameter of the fibers. The scale selected for the evaluation of webs prepared in accordance with the present invention, therefore, is based on producing nonwoven webs by several processes for a variety of applications.

The term "threadline" is used throughout the specification and claims to refer to the shaped article formed as the polymer solution is forced through a die orifice but before such shaped article has solidified or dried. A threadline is essentially liquid or semisolid. The term "fiber" is used to designate the solidified or dried threadline. The transition from a threadline to a fiber is gradual. -

In respect to the "back side" and "front side" of the threadline curtain, the back side of the curtain is the side toward which the moving foraminous surface approaches. The foraminous surface then passes under the threadline curtain and moves away from it with a nonwoven web having been formed thereon. The side where the web has been formed is the front side of the threadline curtain.

Whenever possible, all units are SI units (International System of Units), whether Basic or Derived. Thus, the unit for viscosity is the pascal-second, abbreviated herein as Pa s. The pascal-second is equal to 10 poise, the more common unit of viscosity.

Turning first to the method of a preferred embodiment of the present invention for preparing a substantially improved nonwoven web including superabsorbent fibers, such method generally includes the following steps:
A. preparing an aqueous polymer solution of a linear superabsorbent precursor polymer;
B. extruding the resulting polymer solution through a die having a plurality of orifices to form a plurality of threadlines;
C. attenuating the resulting threadlines with a primary gaseous source;
D. drying the attenuated threadlines with a secondary gaseous source to form fibers;
E. depositing the resulting fibers randomly on a moving foraminous surface to form a substantially uniform web; and
F. insolubilizing the fiber into a water swellable but water insoluble web.

In general, the first two steps are independent of the apparatus or details of the process employed. As will become evident hereinafter, however, this is not the case for the remaining steps. That is, some of the limitations of the attenuating, drying, and depositing steps depend on whether the superabsorbent precursor fibers produced are substantially continuous or continuous.

The first step (step A) of the method involves preparing an aqueous superabsorbent precursor polymer solution which includes from about 10 to about 75 percent by weight of the polymer. Because the solubility of the polymer in water is inversely proportional to the polymer molecular weight, higher concentrations, i.e., concentrations above about 40 percent by weight, are practical only when polymer molecular weights are below about 100,000. The preferred concentration range is from about 20 to about 60 percent by weight. Most preferably, the concentration of superabsorbent precursor polymer in the solution is in the range of from about 25 to about 40 percent by weight.

In general, the superabsorbent precursor polymer of the preferred embodiment has a molecular weight of from about 300,000 to about 10,000,000. The preferred ranges are from about 3,000,000 to about 8,000,000, more preferably from about 500,000 to about 4,000,000.

The superabsorbent precursor polymer solution also can contains, besides a cross linkable moiety in the polymer backbone and/or cross linking agents, minor amounts of other materials, i.e., amounts of other materials that together constitute less than 50 percent by weight of the total solids content of the solution. Such other materials include, by way of illustration only, plasticizers, such as polyethylene glycols, glycerin, and the like; colorants or dyes: extenders, such as clay, starch, and the like; other functional substances; and the like.

In the second step (step B), the polymer solution is extruded at a temperature of from about 20°C to about 180°C and a viscosity at the extrusion temperature of from about 3 to about 1000 Pa s through a die having a plurality of orifices to form a plurality of threadlines, which orifices have diameters in the range of from about 0.20 to about 1.2 mm. The extrusion temperature preferably will be in the range of from about 70°C to about 95°C. The preferred polymer solution viscosity is from about 5 to about 30 Pa s. The orifices in the die preferably will have diameters of from about 0.3 to about 0.6 mm. The orifices are arranged in as many as about 7 multiple rows. Such rows are perpendicular to the direction of travel of the moving foraminous surface upon which the nonwoven web is formed. The length of such rows defines the width of the web which is formed. Such arrangement of orifices results in a "sheet" or "curtain" of threadlines. The thickness of such curtain is determined by the number of rows of orifices, but it is very small in comparison with the width of the curtain. For convenience, such curtain of threadlines occasionally will be referred to herein as the "threadline plane." Such plane is perpendicular to the moving foraminous surface upon which the web is formed, although such an orientation is neither essential nor required.

While solution viscosity is a function of temperature, it also is a function of polymer molecular weight and the concentration of the polymer in the solution. Consequently, all of these variables need to be taken into consideration to maintain the solution viscosity at the extrusion temperature in the proper range.

The resulting threadlines then are attenuated in step C with a primary gaseous source to form fibers under conditions sufficient to permit the viscosity of each threadline, as it leaves a die orifice and for a distance of no more than about 8 cm, to increase incrementally with increasing distance from the die, while maintaining uniformity of viscosity in the radial direction. The rate of threadline attenuation must be sufficient to provide fibers having the desired strength and mean fiber diameter without significant fiber breakage. The primary gaseous source has a relative humidity of from about 40 to 100 percent and a temperature of from about 20°C to about 100°C, a horizontal angle of incidence of from about 70° to about 110°, and a vertical angle of incidence of no more than about 90°.

When substantially continuous fibers are being formed, the velocity of the primary gaseous source is in the range of from about 150 to about 400 m/s. The more preferred primary gaseous source velocity is from about 60 to about 300 m/s. The primary gaseous source velocity most preferably is in the range of from about 70 to about 200 m/s. For the production of continuous fibers, however, the velocity of the primary gaseous source is in the range of from about 30 to about 150 m/s.

The attenuation step involves a balance between attenuating aspects and drying aspects since some loss of water from the threadlines usually is inevitable. However, optimum attenuating conditions may not always coincide with optimum drying conditions. Consequently, a conflict between the two parameters may arise which requires finding a compromise set of conditions.

It is important that the threadlines be attenuated to the desired level without breakage. An excessive attenuation rate creates excessive stress on the threadlines which leads to frequent threadline or fiber breaks and increased shot formation, particularly with microfibers having diameters in the range of from about 0.1 to about 10 µm. Too slow an attenuation rate, though, fails to give sufficiently strong fibers. On the other hand, too rapid threadline drying, especially during the attenuation step, results in increased breaks and increased shot production. If threadline drying is too slow during the drying step, excessive interfiber bonding or fusing occurs as a result of the fibers being too wet as they are laid down on the moving foraminous surface. Consequently, ideal drying conditions typically are not optimum for the production of highly attenuated, strong fibers. Thus, the somewhat opposing requirements for attenuating and drying the threadlines are accomplished by controlling the relative humidity and temperature of the primary gaseous source, as well as its velocity. The attenuating step results in no more than partial drying of the threadlines to provide the required incremental increase in threadline viscosity.

Drying of the attenuated and partially dried threadlines is accomplished in step D by means of a secondary gaseous source. The secondary gaseous source has a temperature of from about 140°C to about 320°C. The vertical and horizontal angle of incidence requirements are the same as those for the primary gaseous source. For substantially continuous fiber production, the secondary gaseous source has a velocity of from about 60 to about 125 m/s. The production of continuous fibers requires a secondary gaseous source having a velocity of from about 30 to about 150 m/s.

As used herein, the term "primary gaseous source" means a gaseous source which is the first to contact the threadlines upon emergence from the die. The term "secondary gaseous source" refers to a gaseous source which contacts the threadlines or fibers after the threadlines have been contacted by the primary gaseous source. Thus, "primary" and "secondary" refer to the order in which two gaseous sources contact the threadlines after they have emerged from the die. Subsequent gaseous sources, if used, would be referred to as "tertiary," "quaternary," and so forth. Although coming within the scope of the present invention, the use of such subsequent gaseous sources usually is neither practical nor necessary and, consequently, is not preferred, with two exceptions which will be described later.

Each of the gaseous sources required by steps C and D, and each additional gaseous source, if used, preferably will comprise at least two gaseous streams, with two streams being more preferred. When two streams are employed, they are located on opposite sides of the threadline curtain or plane. The stream impinging the filaments from the front side of the threadline curtain has a positive vertical angle of incidence, whereas the stream impinging the filaments from the back side of the threadline curtain has a negative vertical angle of incidence. However, the absolute value of the vertical angle of incidence for each stream must be within the limitations described herein, although both streams need not have the same absolute value for their vertical angles of incidence. Consequently, it should be understood that the requirement with respect to the vertical angle of incidence refers to an absolute value when a gaseous source involves more than one gaseous stream.

In the last step of the method of the preferred embodiment, step E, the fibers resulting from the previous step are deposited randomly on a moving foraminous surface. In the case of substantially continuous fiber production, the moving foraminous surface is from about 10 to about 60 cm from the opening from which the last gaseous source to contact the threadlines emerges. The distance between the moving foraminous surface and such opening on occasion is referred to herein as the forming distance. The mean fiber diameter is in the range of from about 0.1 to about 10 µm. The fibers are substantially uniform in diameter and are substantially free of shot.

When continuous fibers are produced, the forming distance preferably is from about 10 to about 100 cm, and the mean fiber diameter is in the range of from about 10 to about 100 µm. The continuous fibers produce a substantially uniform web.

The area or scale used for comparison purposes in evaluating web uniformity is a function of fiber diameter. The scale for a web including substantially continuous fibers is in the range of from about 0.4 to about 1.9 cm², while the scale for a web including continuous fibers is in the range of from about 1.9 to about 6.5 cm².

Step C requires controlled macro scale turbulence and conditions sufficient to permit the viscosity of each threadline, as it leaves a die orifice, to increase incrementally with increasing distance from the die, while maintaining uniformity of viscosity in the radial direction, at a rate sufficient to provide fibers having the desired attenuation and mean fiber diameter without significant fiber breakage. The means for meeting both requirements involves controlling four parameters or variables associated with the gaseous source, including relative humidity, temperature, velocity, and orientation relative to the threadline curtain. Macro scale turbulence primarily is a function of gaseous stream velocity and the orientation of the gaseous source as it impinges the threadline curtain. The viscosity of the threadline, although affected by gaseous source velocity, is a function of the relative humidity and temperature of the primary gaseous source. Such parameters or variables are discussed below in respect to "Macro Scale Turbulence" and "Threadline Viscosity."

Referring now to Macro Scale Turbulence, attenuating and drying are carried out under conditions of controlled macro scale turbulence. In a preferred embodiment, attenuating and drying are carried out under conditions of minimal macro scale turbulence, thereby assisting the formation of a web which is substantially uniform. As used herein, the term "minimal macro scale turbulence" means only that degree of turbulence which will permit the desired uniform web formation to occur which is in part dependent on uniform fiber spacing and orientation.

Some turbulence is unavoidable, indeed necessary, given the fact that attenuation results from the entrainment of threadlines in a moving gaseous stream. A minimum gaseous stream velocity is determined empirically..The minimum gaseous source velocity is much higher than the extrusion velocity.

In certain instances, macro scale turbulence is greater than minimal, although still controlled. For example, when fibers or particles are to be commingled with the threadlines as they are formed, a greater degree of turbulence is required to achieve a degree of commingling which is sufficient to provide a coherent uniform web.

Macro scale turbulence also is a function of the nature of the gaseous source and its orientation as it impinges the threadline curtain. In addition, the efficiency of threadline attenuation is, at least in part, dependent upon gaseous source orientation. Gaseous source orientation is defined by the horizontal angle of incidence and the vertical angle of incidence.

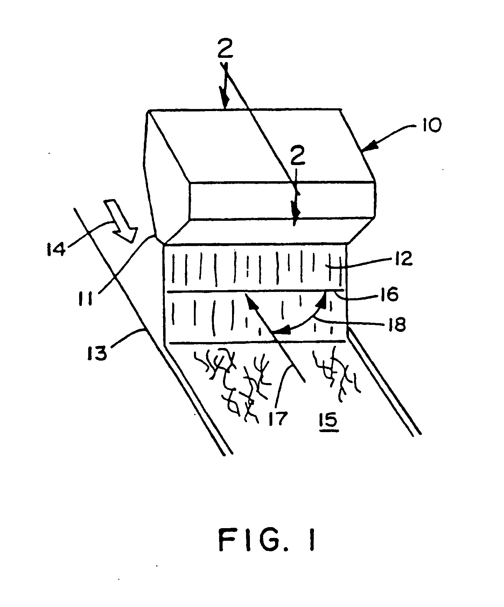

The horizontal angle of incidence is best defined with reference to Figure 1. Figure 1 is a perspective schematic view partially illustrating the preparation of a nonwoven in accordance with one embodiment of the present invention. Polymer solution is extruded through a plurality of orifices in face 11 of die 10 to form threadline curtain 12. As threadline curtain 12 meets foraminous belt 13 moving in the direction of arrow 14, nonwoven web 15 is formed. Line 16 lies in the plane of threadline curtain 12 and is parallel with face 11 of die 10. Arrow 17 represents the orientation of a gaseous stream relative to line 16, with the direction of flow being in the same direction as arrow 17. Angle 18 formed by line 16 and arrow 17 is the horizontal angle of incidence. Angle 18 is determined relative to the right-hand portion of line 16 with respect to an observer facing die 10, toward whom foraminous belt 13 is moving. The horizontal angle of incidence of each gaseous source is in the range of from about 70° to about 110°, with an angle of about 90° being preferred.

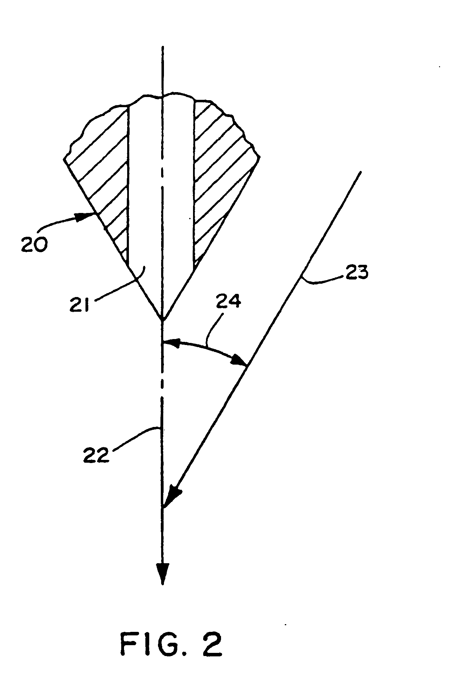

The vertical angle of incidence is best defined with reference to Figure 2. Figure 2 shows a cross-section view of a small portion of die 20 having orifice 21, taken along line 2-2 of Figure 1. Arrow 22 represents the centerline of the threadline (not shown) emerging from orifice 21, with the direction of flow being the same as the direction of arrow 22. Arrow 23 represents the orientation of a gaseous stream relative to arrow 22, with the direction of flow being in the same direction as arrow 23. Angle 24 formed by arrows 21 and 22 is the vertical angle of incidence. The vertical angle of incidence of any gaseous source generally will be no more than about 90°. Preferably, the vertical angle of incidence will be no more than about 60°, and most preferably no more than about 45°. The preferred values for the vertical angle of incidence refer to absolute values when any given gaseous source involves more than one gaseous stream.

Macro scale turbulence is in part a function of the orientation of the gaseous source. From a consideration of Figures 1 and 2, the horizontal angle of incidence has the least effect on macro scale turbulence (i.e., web uniformity) when such angle is about 90°. Similarly, the vertical angle of incidence has the least effect on macro scale turbulence when it is about 0°. As the horizontal angle of incidence deviates from 90° and/or the vertical angle of incidence increases above 0°, macro scale turbulence to some extent is reduced by decreasing the gaseous source velocity.

The macro scale turbulence of any gaseous source needs to be carefully controlled along the entire width of the threadline curtain. Such control in part is accomplished through the use of manifold designs. For example, a manifold is used which has a gradually reduced cross-section. In addition, a combination of honeycomb sections with screens or sintered, porous metal baffles effectively destroys the undesired large scale turbulent eddy currents which may otherwise be formed.

As the controlled high velocity gaseous source exits the opening of a duct or manifold, it entrains the surrounding ambient air, and its velocity is decreased as the distance from such opening increases. During the momentum transfer between the high velocity gaseous source and the ambient air, the size of turbulent eddies increases. Small scale turbulent eddies help entangle the fibers at an early stage near the opening from which the gaseous source emerges, but eddies which grow at distances of around 50 cm or more from such opening adversely affect web uniformity by the formation of heavy and light basis weight areas in the web. It is important that formation distances be kept within the limits specified herein. Moreover, some ambient air entrainment is essential for keeping large scale eddy currents at a minimum.

Referring now to Threadline Viscosity, the primary gaseous source has a relative humidity of from about 30 to 100 percent. More preferably, such gaseous source will have a relative humidity of from about 60 to about 95 percent. Most preferably, the relative humidity of the primary gaseous source will be in the range of from about 60 to about 90 percent.

It has been found that the presence of water droplets in the humidified gaseous source has adverse effects on threadline and fiber formation, particular with respect to the formation of shot. Consequently, it is preferred that any water droplets which may be present in the humidified gaseous source have diameters less than the diameters of the threadlines. Most preferably, the humidified gaseous stream is essentially free of water droplets.

In practice, water droplets are removed successfully from the humidified gaseous source through the use of an impingement separator. Additionally, it is helpful to heat all passageways through which the humidified gaseous source passes prior to impinging the threadlines. However, passageway temperatures should be such that the temperature of the humidified gaseous source remains within acceptable limits as already described.

The temperature of the primary gaseous source is in the range of from about 20°C to about 100°C. Such temperature more preferably is in the range of from about 40°C to about 100°C, and most preferably from about 60°C to about 90°C.

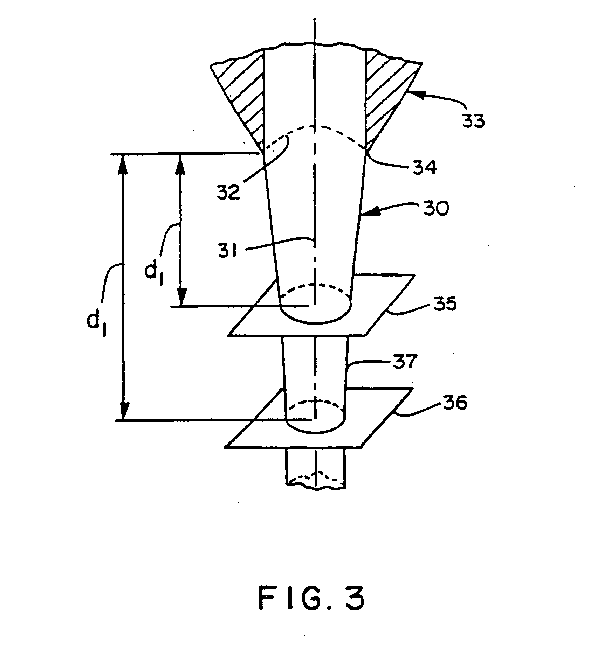

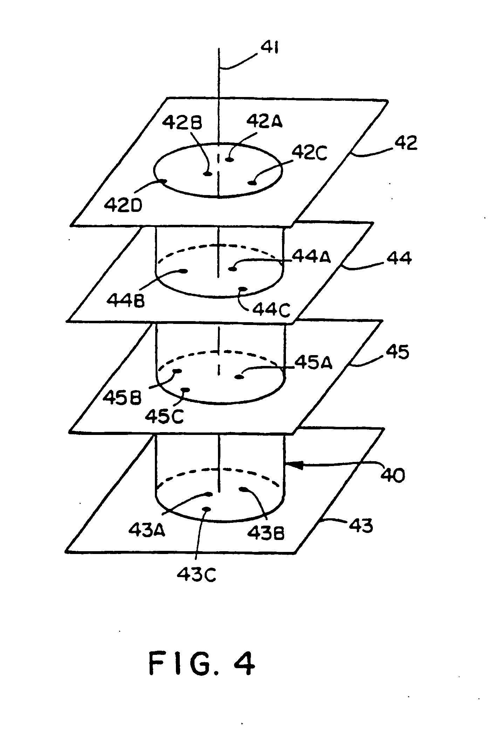

The viscosity requirements are understood with reference to Figures 3 and 4. Figure 3 is a perspective view of a portion of threadline 30 having longitudinal axis 31 as it emerges from orifice 32 in die 33 (shown in partial cross-section) having face 34. Plane 35 is perpendicular to axis 31 and is at a distance d₁ from die face 34. Plane 36 also is perpendicular to axis 31 and is at a distance d₂ from die face 34, with d₂ being greater than d₁ (i.e., d₂ > d₁). Section 37 of threadline 30 lies between planes 35 and 36. Because threadline 30 is being attenuated, the diameter of the threadline decreases with increasing distance from the die. Consequently, section 37 of threadline 30 approximates an inverted truncated cone or, more properly, an inverted frustrum of a cone.

Section 37 of threadline 30 of Figure 3 which is located between planes 35 and 36 of Figure 3 is shown in perspective view in Figure 4. In Figure 4, threadline section 40 has axis 41 and is defined by upper plane 42 (i.e., plane 35 in Figure 3), and lower plane 43 (i.e., plane 36 in Figure 3). Both planes are perpendicular to axis 41 and are parallel with each other. Additional planes 44 and 45 are shown, which planes also are perpendicular to axis 41 (or parallel with planes 42 and 43) and are at distances d₃ and d₄, respectively, from the face of the die which is not shown (i.e., face 34 of die 33 in Figure 3). Upper plane 42 and lower plane 43 are at distances d₁ and d₂, respectively, from the face of the die. Thus, d₁ < d₃ < d₄ < d₂. Points 42A, 42B, 42C, and 42D lie in upper plane 42. Similarly, points 43A, 43B, and 43C lie in lower plane 43; points 44A, 44B, and 44C lie in plane 44; and points 45A, 45B, and 45C lie in plane 45.

With reference to Figure 4, uniformity of viscosity in the radial direction provides that the viscosity of the threadline at any point lying in a plane perpendicular to axis 41 is approximately the same. That is, the viscosity of the threadline at points 42A, 42B, 42C, and 42D is essentially the same. Moreover, the viscosity at points 43A, 43B, and 43C is essentially the same; the viscosity at points 44A, 44B, and 44C is essentially the same; and the viscosity at points 45A, 45B, and 45C is essentially the same.

However, the viscosity of the threadline increases incrementally with increasing distance from the die. That is, the viscosity of the threadline at any of points 44A, 44B, and 44C, again with reference to Figure 4, is greater than the viscosity at any of points 42A, 42B, 42C, and 42D. The viscosity at any of points 45A, 45B, and 45C in turn is greater than the viscosity at any of points 44A, 44B, and 44C. Finally, the viscosity at any of points 43A, 43B, and 43C is greater than the viscosity at any of points 45A, 45B, and 45C.

All of the foregoing viscosity relationships can be expressed mathematically as follows, in which h_{Pn} is the viscosity at point n:
h_{P43A} » h_{P93B} » h_{P43C} > h_{P45A} » h_{P45B} » h_{P45C} > h_{P44A} »
h_{P44B} » h_{P44C} > h_{P42A} » h_{P42B} » h_{P42C} » h_{P42D}

The extent of the increase of viscosity with increasing distance from the die is critical over the distance from the die specified herein. However, the increase should not be so large as to contribute to fiber breakage or so small that the threadline does not solidify sufficiently before reaching the moving foraminous surface on which the nonwoven web is formed. The term "incrementally" is associated with the increase in viscosity to convey the concept that such increase is a slight or imperceptible increase from a given plane having a very small thickness to the next or adjacent plane downstream from the die. Thus, such change in viscosity can be considered to be the derivative dy/dx, where dy is the increase in viscosity resulting from an increase dx in distance from the die when such increase in distance approaches zero.

It is problematic to measure the viscosity of the threadline at any given point, or to measure or estimate the concentration and temperature from which a viscosity could be calculated or estimated. Nevertheless, it has been determined empirically that the foregoing conditions for viscosity must exist when fibers having the required characteristics, including the absence of shot, desired fiber diameters, and desired molecular orientation attenuation are obtained. Significant deviations from such viscosity requirements produce shot, broken fibers, irregular web formation, and/or fibers having highly variable and irregular diameters.

It has been found that fibers or particles can be commingled with the threadlines. Primary and secondary gaseous sources are employed with the fibers or particles being introduced into the secondary gaseous source. When two secondary gaseous streams are employed, which is preferred, the fibers or particles can be included in either or both of the secondary gaseous streams.

Alternatively, three gaseous sources can be employed in the preparation of a coformed web, including a primary gaseous source, a secondary gaseous source, and a tertiary gaseous source. In a first exception to the general avoidance of the use of a subsequent gaseous source, i.e., a gaseous source in addition to primary and secondary gaseous sources, the fibers or particles are included in the tertiary gaseous source, in which case a single tertiary gaseous stream usually is sufficient. When a fiber-carrying or particle-carrying tertiary gaseous source is employed, the tertiary gaseous source will be at ambient temperature and have a velocity of from about 5 to about 15 m/s. While a heated gaseous source can be used, care must be taken to avoid softening the fibers to an extent which causes excessive bonding of the superabsorbent precursor fibers to each other and/or to the fibers or particles with which they are intermingled.

A second exception relates to the formation of a nonwoven web from continuous fibers. In this case, three gaseous sources contribute to the control of turbulence and, consequently, to improved web uniformity. The characteristics of the three gaseous sources are described briefly below.

The primary gaseous source has a relative humidity of from about 40 to 100 percent, a temperature of from about 20°C to about 100°C, a horizontal angle of incidence of from about 70° to about 110°, and a vertical angle of incidence of no more than about 90°. The velocity of the primary gaseous source is no more than about 45 m/s. Such velocity preferably will be in the range of from about 5 to about 15 m/s. The function of the primary gaseous source is to provide the conditions necessary to permit the required threadline viscosity increases as described hereinbefore. The primary gaseous source in this case functions as a conditioning source.

The secondary gaseous source has a temperature of from about 20°C to about 100°C, a horizontal angle of incidence of from about 70° to about 110°, and a vertical angle of incidence of no more than about 90°. The velocity of the secondary gaseous source typically is no more than about 45 m/s. The velocity of the secondary gaseous source is in the range of from about 5 to about 15 m/s. The secondary gaseous source serves to partially dry the threadlines partially, although a small degree of attenuation also may take place.

Finally, the tertiary gaseous source has a lower temperature and a higher velocity than either the primary gaseous source or the secondary gaseous source. The tertiary gaseous source functions to attenuate and more fully dry the fibers. The tertiary gaseous source has a temperature in the range of from about 10°C to about 50°C. The velocity of the tertiary gaseous source ranges from about 30 to about 245 m/s. In addition, such gaseous source has a horizontal angle of incidence of from about 70° to about 110° and a vertical angle of incidence of no more than about 90°.

Preferred embodiments of the present invention is further illustrated by the actual examples which follow. Such examples, however, are not to be construed as in any way limiting either the scope of the present invention.

### Example 1

5.9 kg of acrylic acid, 2.29 kg of sodium hydroxide, 143 g of 3-amino-1-propanol vinyl ether, and 11.97 gram of potassium persulfate, all available from Aldrich Chemical Company, were added into a 10 gallon jacketed reactor containing 21.78 kg of distilled water and equipped with an agitator. The added components were mixed at room temperature to form a completely dissolved solution. The reactor then was heated to 60°C for four hours. The agitator was on continuously. The formed polyacrylic acid sodium salt solution includes 73.8% by weight of sodium acrylate, 24.2% by weight of acrylic acid, and 2% by weight of 3-amino-1-propanol vinyl ether.

### Example 2

The polymer solution prepared in Example 1 was used to prepare nonwoven webs on an apparatus having a six-inch (15.2-cm) wide die having 120 orifices (20 orifices per inch or about 11.8 orifices per cm). Each orifice had a diameter of 0.46 mm. The die was constructed essentially as described in U.S. Patent Nos. 3,755,527, 3, 795, 571, and 3, 849, 241.The primary gaseous source was divided into two streams, the exits of which were located parallel with and closely adjacent to the row of extrusion orifices. Each primary gaseous stream exit was about 0.86 mm in width. The ducts leading to the two primary gaseous stream exits were at an angle of 30° from the vertical, i.e., the plane in which the centers of the extrusion orifices were located. Thus, the vertical angles of incidence for the two primary gaseous streams were 30° and -30°, respectively. The absolute value of the vertical angle of incidence for each of the two primary gaseous streams was 30°. The horizontal angle of incidence for each primary gaseous stream was 90°.

The secondary gaseous source also was divided into two secondary gaseous streams. The first secondary gaseous stream, was introduced on the back side of the threadline curtain. The vertical angle of incidence for the first secondary gaseous stream was -30°. The horizontal angle of incidence was 90°. The exit of the first secondary gaseous stream was located about 5 cm below the die tip and about 2.5 cm from the threadline curtain.

The second secondary gaseous stream was introduced on the front side of the threadline curtain. The vertical angle of incidence for the second secondary gaseous stream was about 0° and the horizontal angle of incidence was 90°. Thus, the second secondary gaseous stream exited the secondary gaseous stream conduit approximately parallel with the threadline curtain. The exit of the second secondary gaseous stream was located about 5 cm below the die tip and about 10 cm from the threadline curtain. The moving foraminous surface was located roughly 22-76 cm below the secondary gaseous source exits which were approximately equal distances below the die tip. A vacuum of 2-6 inches (0.005-0.015 atm) water was maintained under the wire.

The sodium polyacrylic acid copolymer solution of Example 1 (26% solids) was heated in a two-liter Buchi autoclave at 50°C under air pressure at 80 psig (5.4 atm).

The solution was pumped by means of a Zenith metering pump to the die through a transfer line heated at about 82°C. The solution was extruded at about 82°C. The primary gaseous source was hot humidified air at a temperature of approximately 93°C, 79% RH and a pressure of 6 psig (0.41 atm) before the outlet of the primary air gap. The secondary gaseous source was compressed-air heated to a temperature of 260°-316°C; the flow rate was 300-400 cfm (42.5-61.4 liters per second). The die tip temperature was maintained at 82°C, and the extrusion rate was 0.33 - 0.83 g per minute per orifice.

Four different solutions extrusion rates, 0.33, 0.55, 0.67, and 0.83 g per min, were employed to form nonwoven webs. The basis weight for each web produced ranged from 34 to 38 g per square meter. Fiber size distribution measurements were made on these four webs. The fiber size distribution measurements involved measuring the diameter of each fiber which crossed an arbitrary straight line drawn on a scanning electron micrograph and typically required measuring the diameters of 50 fibers. The results of such measurements are summarized in Table 2-1.

**Table 2-1**

| **Fiber Diameter Distribution** | | | | |
|---|---|---|---|---|
| | % Frequency | | | |
| Web Number | 1 | 2 | 3 | 4 |
| Throughput (gpm) | 100 | 80 | 60 | 40 |
| Fiber size (µm) | | | | |
| 1.5 | 0 | 0 | 4 | 4 |
| 2 | 0 | 0 | 6 | 8 |
| 2.5 | 0 | 0 | 2 | 18 |
| 3 | 0 | 0 | 10 | 4 |
| 3.5 | 6 | 2 | 4 | 18 |
| 4 | 10 | 6 | 6 | 2 |
| 4.5 | 8 | 16 | 6 | 6 |
| 5 | 16 | 18 | 6 | 6 |
| 5.5 | 6 | 4 | 4 | 6 |
| 6 | 16 | 12 | 14 | 8 |
| 6.5 | 8 | 16 | 12 | 10 |
| 7 | 0 | 2 | 2 | 2 |
| 7.5 | 8 | 12 | 4 | 2 |
| 8 | 8 | 4 | 2 | 0 |
| 8.5 | 0 | 6 | 12 | 4 |
| 9 | 2 | 0 | 0 | 2 |
| 9.5 | 6 | 0 | 0 | 0 |
| 10 | 2 | 2 | 0 | 0 |
| 10.5 | 2 | 0 | 2 | 0 |
| 11 | 0 | 2 | 0 | 0 |
| 11.5 | 0 | 0 | 0 | 0 |
| 12 | 0 | 0 | 2 | 0 |
| More | 2 | 0 | 2 | 0 |
| AVERAGE | 6.015 | 5.8308 | 5.3928 | 4.1154 |
| STD DEV | 2.06286 | 1.608323 | 2.53919 | 1.987291 |

The data from Table 2-1 were plotted as frequency versus fiber diameter in µm to aid in the visualization of the fiber diameter frequencies.

The tensile properties of the nonwoven webs obtained were measured in accordance with standard test procedures, Federal Standard 191A, Method 5102. The strip tensile procedure gave results for peak load, percent elongation, and energy.

The tensile characteristics of the nonwoven webs were obtained. All reported values were normalized to allow for differences in basis weights.

To assist in the visualization of the tensile characteristics data, the data were plotted as bar graphs, with separate bars for MD data, CD data, and the average of the MD and CD data, respectively.

### Example 3

In order to prepare a coformed web, the procedure of Example 2 was essentially repeated. A largely softwood pulp sheet (Coosa CR-54, manufactured by Kimberly-Clark Corporation at its Coosa Pines, Alabama, Mill) was fiberized with a hammer mill and then blown with air at a velocity of 83 m/s through a rectangular duct having a depth of 2.5 cm. The dilution rate, defined as g of fiberized pulp per cubic meter of carrier air volume, was kept in the range of from about 2.8 to about 8.5 to minimize flocculation. The resulting air-borne fiber stream then was injected into the threadline-carrying first secondary gaseous stream at the region where the threadline-carrying first secondary gaseous stream and second secondary gaseous stream met. Both the vertical and horizontal angles of incidence of the air-borne fiber stream were about 90°. The stream exited the rectangular duct about 10 cm from the region where the two secondary gaseous streams met.

In each case, the resulting coformed web was well integrated and strong, but soft, bulky, and absorbent. The web was composed of 50-70 percent by weight of pulp fibers and had a basis weight of about 500 g/m². Even after heat treatment in a convection oven to cross link sodium polyacrylic acid copolymer, these webs were very soft, absorbent and of reasonable mechanical strength as shown in Table 3.1. Such coformed webs are useful as wipes or as components of other absorbent products.

| **Peak Tensile Property** | | | | |
|---|---|---|---|---|
| Web Number | 1 | 2 | 3 | 4 |
| SAF Comp (%) | 71 | 66 | 60 | 50 |
| Load (m) | 392 | 349 | 424 | 406 |
| Strain (%) | 11.9 | 14.0 | 16.6 | 9.5 |
| Energy (m) | 2.59 | 3.04 | 4.25 | 2.40 |

### Example 4

In this example, in addition to Coosa pulp, superabsorbent powder (Favor 880 from Stockhausen, Inc.) was introduced into the pulp stream prior to its meeting the threadline-carrying first secondary gaseous stream. The composition was about 33% superabsorbent fiber, 33% pulp, and 34% superabsorbent powder. The total basis weight was measured. This material was quite soft after been made. In 30 minutes, it wicked 0.9% NaCl water solution to about 23 cm.

### Example 5

This example is similar to Example 4, with the exception of material composition. A nonwoven coform web was successfully made with about 3% superabsorbent fiber, 3% Coosa pulp, and about 94% superabsorbent powder (Favor 880 from Stockhausen, Inc.). The material had excellent SAM superabsorbent material containment capability since fair amount of superabsorbent powder particles were adhered to the superabsorbent fibers.

### Example 6

This is similar to Example 2, with the exception that the relative humidity of the primary gaseous stream was varied. As determined from SEM, satisfactory results were achieved only when the relative humidity level is in the range of 30% to 100%.

Having thus described the invention, numerous changes and modifications thereof will be readily apparent to those having ordinary skill in the art without departing from the scope of the invention.

## Claims

1. A method of preparing a nonwoven web having substantially continuous superabsorbent fine fiber, comprising the steps of:
preparing an aqueous polymer solution of 10 to 75 percent by weight of a linear superabsorbent precursor polymer; and
extruding said polymer solution through a die (10) having a plurality of orifices (21) to form a plurality of threadlines, said orifices having diameters in the range of from 0.20 to 1.2 mm;
**characterised by**:
said precursor polymer having a molecular weight of from 300,000 to 10,000,000;
said polymer solution being extruded at a temperature of from 20°C to 180°C and a viscosity of from 3 to 1000 Pa sec; and
attenuating said threadlines with a primary gaseous source under conditions sufficient to permit the viscosity of each threadline, as it leaves a die orifice (21) and for a distance of no more than 8 cm, to increase incrementally with increasing distance from the die (10), while substantially maintaining uniformity of viscosity in the radial direction, at a rate sufficient to provide fibers having the desired attenuation and mean fiber diameter without significant fiber breakage;
wherein said primary gaseous source has a relative humidity of from 30 to 100 percent;
wherein said primary gaseous source has a temperature of from 20°C to 100°C, a horizontal angle of incidence of from 70° to 110°, and one of:
(i) a velocity of less than 30 m/s and a vertical angle of incidence of about 90°; and
(ii) a velocity of from 30 m/s to 400 m/s and a vertical angle of incidence of no more than 90°.

2. A method of preparing a nonwoven web having substantially continuous superabsorbent fine fiber as claimed in Claim 1, wherein said primary gaseous source has a relative humidity of from 60 to 95 percent.

3. A method of preparing a nonwoven web having substantially continuous super absorbent fine fiber as claimed in Claim 1, wherein said primary gaseous source has a relative humidity of from 65 to 90 percent.

4. A method of preparing a nonwoven web having substantially continuous superabsorbent fine fiber as claimed in Claim 1, wherein the velocity of said primary gaseous source is from 150 m/s to 400 m/s and wherein said vertical angle of incidence is no more than 90°, and further comprising:
d. drying said threadlines to form fibers with a secondary gaseous source at a temperature of from 140°C to 320°C, and having a velocity of from 60 to 125 m/s, which secondary gaseous source has a horizontal angle of incidence of from 70° to 110°, and a vertical angle of incidence of no more than 90°.

5. A method of preparing a nonwoven web having substantially continuous superabsorbent fine fiber as claimed in any preceding claim, further comprising:
e. depositing the fibers randomly on a moving foraminous surface (13) to form a substantially uniform web on a scale of from 0.4 to 1.9 cm², said moving foraminous surface being from 10 to 60 cm from the opening from which the last gaseous source to contact the threadlines emerges, which fibers have a mean fiber diameter in the range of from 0.1 to 10 µm and are substantially free of shot; in which said attenuating and drying steps are carried out under conditions of controlled macro scale turbulence and said fibers are of a length such that they can be regarded as continuous in comparison with their diameters.

6. A method of preparing a nonwoven web having substantially continuous superabsorbent fine fiber as claimed in Claim 5, further comprising:
f. exposing said uniform web to a high energy source selected from the group consisting of heat, electron beam, microwave, and radio frequency irradiation to render a stable cross-link in the superabsorbent precursor polymer.

7. A method of preparing a nonwoven web having substantially continuous superabsorbent fine fiber as claimed in Claim 5 or 6, further comprising:
g. post treating the stabilized web by humidifying, compacting, embossing, bonding, or laminating, or a combination thereof.

8. A method of preparing a nonwoven web having substantially continuous superabsorbent fine fiber as claimed in Claim 1, wherein the velocity of said primary gaseous source is from 30 m/s to 150 m/s and wherein said vertical angle of incidence is no more than 90° and further comprising:
d. drying said threadlines to form fibers with a secondary gaseous source at a temperature of from 140°C to 320°C and having a velocity of from 30 to 150 m/s, which secondary gaseous source has a horizontal angle of incidence of from 70° to 110°, and a vertical angle of incidence of no more than 90°.

9. A method of preparing a nonwoven web having substantially continuous superabsorbent fine fiber as claimed in Claim 8, further comprising:
e. depositing the fibers randomly on a moving foraminous surface (13) to form a substantially uniform web on a scale of from 1.9 to 6.5 cm², said moving foraminous surface being from 10 to 100 cm from the opening from which the last gaseous source to contact the threadlines emerges, which fibers have a mean fiber diameter in the range of from 10 to 30 *µ*m and are substantially uniform in diameter; in which said attenuating and drying steps are carried out under conditions of minimal macro scale turbulence.

10. A method of preparing a nonwoven web having substantially continuous superabsorbent fine fiber as claimed in Claim 9, further comprising:
f. exposing said uniform web to a high energy source selected from the group consisting of heat, electron beam, microwave, and radio frequency irradiation to render a stable cross-link in the superabsorbent precursor polymer.

11. A method of preparing a nonwoven web having substantially continuous superabsorbent fine fiber as claimed in Claim 9 or 10, further comprising:
g. post treating the stabilized web by humidifying, compacting, embossing, bonding, or laminating, or a combination thereof.

12. A method of preparing a nonwoven web having substantially continuous superabsorbent fine fiber as claimed in Claim 1, wherein the velocity of said primary gaseous source is less than 30 m/s and wherein said vertical angle of incidence is about 90° and further comprising:
d. drying said threadlines to form fibers with a secondary gaseous source at a temperature of from 140°C to 320°C and having a velocity of less than 30 m/s, which secondary gaseous source has a horizontal angle of incidence of from 70° to 110°, and a vertical angle of incidence of no more than 90°.

13. A method of preparing a nonwoven web having substantially continuous superabsorbent fine fiber as claimed in Claim 4, 8 or 12, further comprising:
e. attenuating said fibers with a tertiary gaseous source having a temperature of from 10°C to 50°C, a velocity of from 30 to 240 m/s, a horizontal angle of incidence of from 70° to 110°, and a vertical angle of incidence of no more than 90°.

14. A method of preparing a nonwoven web having substantially continuous superabsorbent fine fiber as claimed in Claim 13, further comprising:
f. depositing the fibers randomly on a moving foraminous surface (13) to form a substantially uniform web on a scale of from 1.9 to 6.5 cm², said moving foraminous surface being from 10 to 100 cm from the opening from which the last gaseous source to contact the threadlines emerges, which fibers have a mean fiber diameter in the range of from 10 to 30 *µ*m and are substantially uniform in diameter; in which said attenuating and drying steps are carried out under conditions of minimal macro scale turbulence.

15. A method of preparing a nonwoven web having substantially continuous superabsorbent fine fiber as claimed in Claim 14, further comprising:
g. exposing said uniform web to a high energy source selected from the group consisting of heat, electron beam, microwave, and radio frequency irradiation to render a stable cross-link in the superabsorbent precursor polymer.

16. A method of preparing a nonwoven web having substantially continuous superabsorbent fine fiber as claimed in Claim 14 or 15, further comprising:
h. post treating the stabilized web by humidifying, compacting, embossing, bonding, or laminating, or a combination thereof.

## Patentansprüche

1. Verfahren zur Herstellung einer vliesartigen Bahn mit einer im Wesentlichen kontinuierlichen hochabsorbierenden Feinfaser, umfassend Schritte des:
Herstellens einer wässrigen Polymerlösung mit 10 Gew.-% bis 75 Gew.-% eines linearen hochabsorbierenden Vorgängerpolymers; und
Extrudierens der Polymerlösung durch eine Düse (10) mit einer Mehrzahl von Öffnungen (21) zur Bildung einer Mehrzahl von Fadengebilden, wobei die Öffnungen Durchmesser in einem Bereich zwischen 0,20 mm und 1,2 mm aufweisen;
**dadurch gekennzeichnet, dass**:
das Vorgängerpolymer ein Molekulargewicht zwischen 300.000 und 10.000.000 aufweist;
die Polymerlösung bei einer Temperatur zwischen 20 °C und 180 °C und einer Viskosität zwischen 3 Pa sec und 1.000 Pa sec extrudiert wird; und durch einen Schritt des
Raffinierens der Fadengebilde mit einer primären Gasquelle unter Bedingungen, die dafür ausreichend sind, dass die Viskosität jedes Fadengebildes bei Verlassen einer Düsenöffnung (21) und für einen Abstand von nicht mehr als 8 cm mit zunehmendem Abstand von der Düse (10) inkrementell zunimmt, während die Gleichmäßigkeit der Viskosität in radialer Richtung im Wesentlichen erhalten bleibt, bei einer Rate, die dafür ausreichend ist, dass Fasern erzeugt werden, die die gewünschte Raffinierung und einen durchschnittlichen Faserdurchmesser aufweisen, ohne dass merkliche Faserrisse auftreten;
wobei die primäre Gasquelle eine relative Feuchtigkeit zwischen 30% und 100% aufweist;
wobei die primäre Gasquelle eine Temperatur zwischen 20 °C und 100 °C, einen horizontalen Einfallswinkel zwischen 70° und 110° und entweder
(i) eine Geschwindigkeit von weniger als 30 m/s und einen vertikalen Einfallswinkel von etwa 90° oder
(ii) eine Geschwindigkeit zwischen 30 m/s und 400 m/s und einen vertikalen Einfallswinkel von nicht mehr als 90° aufweist.

2. Verfahren nach Anspruch 1 zur Herstellung einer vliesartigen Bahn mit einer im Wesentlichen kontinuierlichen hochabsorbierenden Feinfaser, bei dem die primäre Gasquelle eine relative Feuchtigkeit zwischen 60% und 95% aufweist.

3. Verfahren nach Anspruch 1 zur Herstellung einer vliesartigen Bahn mit einer im Wesentlichen kontinuierlichen hochabsorbierenden Feinfaser, bei dem die primäre Gasquelle eine relative Feuchtigkeit zwischen 65% und 90% aufweist.

4. Verfahren nach Anspruch 1 zur Herstellung einer vliesartigen Bahn mit einer im Wesentlichen kontinuierlichen hochabsorbierenden Feinfaser, bei dem die Geschwindigkeit der primären Gasquelle zwischen 150 m/s und 400 m/s liegt, und bei dem der vertikale Einfallswinkel nicht größer als 90° ist, des Weiteren umfassend:
(d) das Trocknen der Fadengebilde zur Bildung von Fasern mit einer sekundären Gasquelle bei einer Temperatur zwischen 140 °C und 320 °C und mit einer Geschwindigkeit zwischen 60 m/s und 125 m/s, wobei die sekundäre Gasquelle einen horizontalen Einfallswinkel zwischen 70° und 110° und einen vertikalen Einfallswinkel von nicht mehr als 90° aufweist.

5. Verfahren nach einem der vorhergehenden Ansprüche zur Herstellung einer vliesartigen Bahn mit einer im Wesentlichen kontinuierlichen hochabsorbierenden Feinfaser, des Weiteren umfassend:
(e) das willkürliche Aufbringen der Fasern auf eine sich bewegende löchrige Oberfläche (13) zur Bildung einer im Wesentlichen gleichmäßigen Bahn mit einer Größe zwischen 0,4 cm² und 1,9 cm², wobei die sich bewegende löchrige Oberfläche zwischen 10 cm und 60 cm von der Austrittsöffnung der letzten Gasquelle zur Kontaktierung der Fadengebilde beabstandet ist, die Fasern einen durchschnittlichen Faserdurchmesser in einem Bereich zwischen 0,1 µm und 10 µm aufweisen und im Wesentlichen schussfrei sind, die Schritte des Raffinierens und Trocknens unter Bedingungen einer kontrollierten hochgradigen Turbulenz vorgenommen werden, und die Fasern eine derartige Länge aufweisen, dass sie im Vergleich zu ihren Durchmessern als kontinuierlich angesehen werden können.

6. Verfahren nach Anspruch 5 zur Herstellung einer vliesartigen Bahn mit einer im Wesentlichen kontinuierlichen hochabsorbierenden Feinfaser, des Weiteren umfassend:
(f) das Bestrahlen der gleichmäßigen Bahn mit einer Hochenergiequelle, die aus einer Wärme, Elektronenstrahlen, Mikrowellen und Hochfrequenzstrahlen umfassenden Gruppe ausgewählt ist, zur Erzeugung einer stabilen Vernetzung in dem hochabsorbierenden Vorgängerpolymer.

7. Verfahren nach Anspruch 5 oder 6 zur Herstellung einer vliesartigen Bahn mit einer im Wesentlichen kontinuierlichen hochabsorbierenden Feinfaser, des Weiteren umfassend:
(g) das Nachbehandeln der stabilisierten Bahn mittels Befeuchten, Verdichten, Gaufrieren, Bonden oder Laminieren oder einer Kombination hieraus.

8. Verfahren nach Anspruch 1 zur Herstellung einer vliesartigen Bahn mit einer im Wesentlichen kontinuierlichen hochabsorbierenden Feinfaser, bei dem die Geschwindigkeit der primären Gasquelle zwischen 30 m/s und 150 m/s liegt, und bei dem der vertikale Einfallswinkel nicht größer als 90° ist, des Weiteren umfassend:
(d) das Trocknen der Fadengebilde zur Bildung von Fasern mit einer sekundären Gasquelle bei einer Temperatur zwischen 140°C und 320 °C und mit einer Geschwindigkeit zwischen 30 m/s und 150 m/s, wobei die sekundäre Gasquelle einen horizontalen Einfallswinkel zwischen 70° und 110° und einen vertikalen Einfallswinkel von nicht mehr als 90° aufweist.

9. Verfahren nach Anspruch 8 zur Herstellung einer vliesartigen Bahn mit einer im Wesentlichen kontinuierlichen hochabsorbierenden Feinfaser, des Weiteren umfassend:
(e) das willkürliche Aufbringen der Fasern auf eine sich bewegende löchrige Oberfläche (13) zur Bildung einer im Wesentlichen gleichmäßigen Bahn mit einer Größe zwischen 1,9 cm² und 6,5 cm², wobei die sich bewegende löchrige Oberfläche zwischen 10 cm und 100 cm von der Austrittsöffnung der letzten Gasquelle zur Kontaktierung der Fadengebilde beabstandet ist, die Fasern einen durchschnittlichen Faserdurchmesser in einem Bereich zwischen 10 µm und 30 µm sowie einen im Wesentlichen gleichmäßigen Durchmesser aufweisen, und die Schritte des Raffinierens und Trocknens unter Bedingungen einer minimalen hochgradigen Turbulenz vorgenommen werden.

10. Verfahren nach Anspruch 9 zur Herstellung einer vliesartigen Bahn mit einer im Wesentlichen kontinuierlichen hochabsorbierenden Feinfaser, des Weiteren umfassend:
(f) das Bestrahlen der gleichmäßigen Bahn mit einer Hochenergiequelle, die aus einer Wärme, Elektronenstrahlen, Mikrowellen und Hochfrequenzstrahlen umfassenden Gruppe ausgewählt ist, zur Erzeugung einer stabilen Vernetzung in dem hochabsorbierenden Vorgängerpolymer.

11. Verfahren nach Anspruch 9 oder 10 zur Herstellung einer vliesartigen Bahn mit einer im Wesentlichen kontinuierlichen hochabsorbierenden Feinfaser, des Weiteren umfassend:
(g) das Nachbehandeln der stabilisierten Bahn mittels Befeuchten, Verdichten, Gaufrieren, Bonden oder Laminieren oder einer Kombination hieraus.

12. Verfahren nach Anspruch 1 zur Herstellung einer vliesartigen Bahn mit einer im Wesentlichen kontinuierlichen hochabsorbierenden Feinfaser, bei dem die Geschwindigkeit der primären Gasquelle bei weniger als 30 m/s liegt, und bei dem der vertikale Einfallswinkel etwa 90° ist, des Weiteren umfassend:
(d) das Trocknen der Fadengebilde zur Bildung von Fasern mit einer sekundären Gasquelle bei einer Temperatur zwischen 140 °C und 320 °C und mit einer Geschwindigkeit von weniger als 30 m/s, wobei die sekundäre Gasquelle einen horizontalen Einfallswinkel zwischen 70° und 110° und einen vertikalen Einfallswinkel von nicht mehr als 90° aufweist.

13. Verfahren nach Anspruch 4, 8 oder 12 zur Herstellung einer vliesartigen Bahn mit einer im Wesentlichen kontinuierlichen hochabsorbierenden Feinfaser, des Weiteren umfassend:
(e) das Raffinieren der Fasern mit einer tertiären Gasquelle mit einer Temperatur zwischen 10 °C und 50 °C, einer Geschwindigkeit zwischen 30 m/s und 240 m/s, einem horizontalen Einfallswinkel zwischen 70° und 110° und einem vertikalen Einfallswinkel von nicht mehr als 90°.

14. Verfahren nach Anspruch 13 zur Herstellung einer vliesartigen Bahn mit einer im Wesentlichen kontinuierlichen hochabsorbierenden Feinfaser, des Weiteren umfassend:
(f) das willkürliche Aufbringen der Fasern auf eine sich bewegende löchrige Oberfläche (13) zur Bildung einer im Wesentlichen gleichmäßigen Bahn mit einer Größe zwischen 1,9 cm² und 6,5 cm², wobei die sich bewegende löchrige Oberfläche zwischen 10 cm und 100 cm von der Austrittsöffnung der letzten Gasquelle zur Kontaktierung der Fadengebilde beabstandet ist, die Fasern einen durchschnittlichen Faserdurchmesser in einem Bereich zwischen 10 µm und 30 µm sowie einen im Wesentlichen gleichmäßigen Durchmesser aufweisen, und die Schritte des Raffinierens und Trocknens unter Bedingungen einer minimalen hochgradigen Turbulenz vorgenommen werden.

15. Verfahren nach Anspruch 14 zur Herstellung einer vliesartigen Bahn mit einer im Wesentlichen kontinuierlichen hochabsorbierenden Feinfaser, des Weiteren umfassend:
(g) das Bestrahlen der gleichmäßigen Bahn mit einer Hochenergiequelle, die aus einer Wärme, Elektronenstrahlen, Mikrowellen und Hochfrequenzstrahlen umfassenden Gruppe ausgewählt ist, zur Erzeugung einer stabilen Vernetzung in dem hochabsorbierenden Vorgängerpolymer.

16. Verfahren nach Anspruch 14 oder 15 zur Herstellung einer vliesartigen Bahn mit einer im Wesentlichen kontinuierlichen hochabsorbierenden Feinfaser, des Weiteren umfassend:
(h) das Nachbehandeln der stabilisierten Bahn mittels Befeuchten, Verdichten, Gaufrieren, Bonden oder Laminieren oder einer Kombination hieraus.

## Revendications

1. Procédé de préparation d'un voile non-tissé ayant des fibres fines, superabsorbantes, sensiblement continues, comprenant les étapes suivantes :
la préparation d'une solution polymère aqueuse comprenant de 10 à 75% en poids d'un polymère précurseur, superabsorbant, linéaire ; et
l'extrusion de ladite solution polymère au travers d'une filière (10) ayant une pluralité d'orifices (21) pour former une pluralité de filets, lesdits orifices ayant des diamètres compris dans la gamme allant de 0,20 à 1,2 mm ;
**caractérisé en ce que** :
ledit polymère précurseur a un poids moléculaire compris entre 300 000 et 10 000 000 ;
ladite solution polymère est extrudée à une température comprise entre 20°C et 180°C et à une viscosité comprise entre 3 et 1000 Pa seconde ; et
l'amincissement desdits filets au moyen d'une source de gaz primaire sous des conditions suffisantes pour permettre à la viscosité de chaque filet, tandis qu'il sort d'un orifice de la filière (21), et sur une distance qui n'est pas supérieure à 8 cm, d'augmenter de manière incrémentielle avec l'augmentation de la distance depuis la filière (10), tout en conservant sensiblement une valeur uniforme dans la direction radiale, à une vitesse suffisante pour fournir des fibres ayant l'amincissement et le diamètre moyen de fibre souhaités sans rupture significative de fibre ;
ladite source de gaz primaire a une humidité relative comprise entre 30 et 100% ;
ladite source de gaz primaire a une température comprise entre 20°C et 100°C, un angle d'incidence par rapport à l'horizontale compris entre 70° et 110°, et
(i) soit une vélocité inférieure à 30 m/seconde et un angle d'incidence par rapport à la verticale d'environ 90° ;
(ii) soit une vélocité comprise entre 30 m/seconde et 400 m/seconde et un angle d'incidence par rapport à la verticale qui n'est pas supérieur à 90°.

2. Procédé de préparation d'un voile non-tissé ayant des fibres fines, superabsorbantes, sensiblement continues, comme revendiqué dans la revendication 1, ladite source de gaz primaire ayant une humidité relative comprise entre 60 et 95%.

3. Procédé de préparation d'un voile non-tissé ayant des fibres fines, superabsorbantes, sensiblement continues, comme revendiqué dans la revendication 1, ladite source de gaz primaire ayant une humidité relative comprise entre 65 et 90%.

4. Procédé de préparation d'un voile non-tissé ayant des fibres fines, superabsorbantes, sensiblement continues, comme revendiqué dans la revendication 1, la vélocité de ladite source de gaz primaire étant comprise entre 150 m/seconde et 400 m/seconde et ledit angle d'incidence par rapport à la verticale n'étant pas supérieur à 90°, et comprenant, en outre :
d. le séchage desdits filets pour former des fibres au moyen d'une source de gaz secondaire à une température comprise entre 140°C et 320°C et ayant une vélocité comprise entre 60 et 125 m/seconde, ladite source de gaz secondaire ayant un angle d'incidence par rapport à l'horizontale compris entre 70° et 110°, et un angle d'incidence par rapport à la verticale qui n'est pas supérieur à 90°.

5. Procédé de préparation d'un voile non-tissé ayant des fibres fines, superabsorbantes, sensiblement continues, comme revendiqué dans l'une quelconque des revendications précédentes, comprenant, en outre :
e. le dépôt aléatoire des fibres sur une surface poreuse (13) en mouvement pour former un voile sensiblement uniforme sur une surface comprise entre 0,4 et 1,9 cm², ladite surface poreuse en mouvement étant à une distance comprise entre 10 et 60 cm depuis l'ouverture à partir de laquelle émerge la dernière source de gaz venant au contact des filets, lesquelles fibres ont un diamètre moyen de fibre compris dans la gamme allant de 0,1 à 10 µm et sont sensiblement dépourvues de variation ; lesdites étapes d'amincissement et de séchage étant réalisées sous des conditions de turbulence contrôlée, à l'échelle macroscopique, et lesdites fibres étant d'une longueur telle qu'elles peuvent être considérées comme continues par rapport à leur diamètre.

6. Procédé de préparation d'un voile non-tissé ayant des fibres fines, superabsorbantes, sensiblement continues, comme revendiqué dans la revendication 5, comprenant, en outre :
f. l'exposition dudit voile uniforme à une source de haute énergie sélectionnée dans le groupe consistant en la chaleur, un faisceau d'électrons, des micro-ondes et une irradiation par radio fréquence pour permettre une réticulation stable dans le polymère précurseur superabsorbant.

7. Procédé de préparation d'un voile non-tissé ayant des fibres fines, superabsorbantes, sensiblement continues, comme revendiqué dans la revendication 5 ou 6, comprenant, en outre :
g. le traitement postérieur du voile stabilisé, par humidification, compactage, gaufrage, liaison ou lamination, ou une combinaison de ceux-ci.

8. Procédé de préparation d'un voile non-tissé ayant des fibres fines, superabsorbantes, sensiblement continues, comme revendiqué dans la revendication 1, la vélocité de ladite source de gaz primaire étant comprise entre 30 m/seconde et 150 m/seconde et ledit angle d'incidence par rapport à la verticale n'étant pas supérieur à 90° et comprenant, en outre :
d. le séchage desdits filets pour former des fibres au moyen d'une source de gaz secondaire à une température comprise entre 140°C et 320°C et ayant une vélocité comprise entre 30 et 150 m/seconde, laquelle source de gaz secondaire ayant un angle d'incidence par rapport à l'horizontale compris entre 70° et 110°, et un angle d'incidence par rapport à la verticale qui n'est pas supérieur à 90°.

9. Procédé de préparation d'un voile non-tissé ayant des fibres fines, superabsorbantes, sensiblement continues, comme revendiqué dans la revendication 8, comprenant, en outre :
e. le dépôt aléatoire des fibres sur une surface poreuse (13) en mouvement pour former un voile sensiblement uniforme sur une surface comprise entre 1,9 et 6,5 cm², ladite surface poreuse en mouvement étant à une distance comprise entre 10 et 100 cm depuis l'ouverture à partir de laquelle émerge la dernière source de gaz venant au contact des filets, lesquelles fibres ont un diamètre moyen de fibre compris dans la gamme allant de 10 à 30 µm et ont un diamètre sensiblement uniforme ; lesdites étapes d'amincissement et de séchage étant réalisées sous des conditions de turbulence minimale à l'échelle macroscopique.

10. Procédé de préparation d'un voile non-tissé ayant des fibres fines, superabsorbantes, sensiblement continues, comme revendiqué dans la revendication 9, comprenant, en outre :
f. l'exposition dudit voile uniforme à une source de haute énergie sélectionnée dans le groupe consistant en la chaleur, un faisceau d'électrons, des micro-ondes et une irradiation par radio fréquence pour permettre une réticulation stable dans le polymère précurseur superabsorbant.

11. Procédé de préparation d'un voile non-tissé ayant des fibres fines, superabsorbantes, sensiblement continues, comme revendiqué dans la revendication 9 ou 10, comprenant, en outre :
g. le traitement postérieur du voile stabilisé, par humidification, compactage, gaufrage, liaison ou lamination, ou une combinaison de ceux-ci.

12. Procédé de préparation d'un voile non-tissé ayant des fibres fines, superabsorbantes, sensiblement continues, comme revendiqué dans la revendication 1, la vélocité de ladite source de gaz primaire étant inférieure à 30 m/seconde et ledit angle d'incidence par rapport à la verticale étant d'environ 90° et comprenant, en outre :
d. le séchage desdits filets pour former des fibres au moyen d'une source de gaz secondaire à une température comprise entre 140°C et 320°C et ayant une vélocité inférieure à 30 m/seconde, laquelle source de gaz secondaire a un angle d'incidence par rapport à l'horizontale compris entre 70° et 110°, et un angle d'incidence par rapport à la verticale qui n'est pas supérieur à 90°.

13. Procédé de préparation d'un voile non-tissé ayant des fibres fines, superabsorbantes, sensiblement continues, comme revendiqué dans la revendication 4, 8 ou 12, comprenant, en outre :
e. l'amincissement desdites fibres au moyen d'une source de gaz tertiaire ayant une température comprise entre 10°C et 50°C, une vélocité comprise entre 30 et 240 m/seconde, un angle d'incidence par rapport à l'horizontale compris entre 70° et 110° et un angle d'incidence par rapport à la verticale qui n'est pas supérieur à 90°.

14. Procédé de préparation d'un voile non-tissé ayant des fibres fines, superabsorbantes, sensiblement continues, comme revendiqué dans la revendication 13, comprenant, en outre :
f. le dépôt aléatoire des fibres sur une surface poreuse (13) en mouvement pour former un voile, sensiblement uniforme sur une surface comprise entre 1,9 et 6,5 cm², ladite surface poreuse en mouvement étant à une distance comprise entre 10 et 100 cm depuis l'ouverture à partir de laquelle émerge la dernière source de gaz venant au contact des filets, lesquelles fibres ont un diamètre moyen de fibre compris dans la gamme allant de 10 à 30 *µ*m et ont un diamètre sensiblement uniforme, lesdites étapes d'amincissement et de séchage étant réalisées sous des conditions de turbulence minimale à l'échelle macroscopique.

15. Procédé de préparation d'un voile non-tissé ayant des fibres fines, superabsorbantes, sensiblement continues, comme revendiqué dans la revendication 14, comprenant, en outre :
g. l'exposition dudit voile uniforme à une source de haute énergie sélectionnée dans le groupe consistant en la chaleur, un faisceau d'électrons, des micro-ondes et une irradiation par radio fréquence pour permettre une réticulation stable dans le polymère précurseur superabsorbant.

16. Procédé de préparation d'un voile non-tissé ayant des fibres fines, superabsorbantes, sensiblement continues, comme revendiqué dans la revendication 14 ou 15, comprenant, en outre :
h. le traitement postérieur du voile stabilisé, par humidification, compactage, gaufrage, liaison ou lamination, ou une combinaison de ceux-ci.
